# EUROPÄISCHE PATENTSCHRIFT

(11) **EP 1 969 129 B1**
(45) Veröffentlichungstag und Bekanntmachung des Hinweises auf die Patenterteilung: **11.03.2009**
(21) Anmeldenummer: 06830713.1
(22) Anmeldetag: 19.12.2006
(51) Int. Cl.: C12P 13/02, C12P 41/00, C07C 211/03, C07C 235/00

(54) **VERFAHREN ZUR HERSTELLUNG VON (R)- UND (S)-4-(1-AMINOMETHYL)BENZOESÄUREMETHYLESTER-SULFAT AUS RACEMISCHEM 4-(1-AMIN0ETHYL)BENZOESÄUREMETHYLESTER**
PROCESS FOR THE PREPARATION OF (R)- AND (S)-4-(1-AMINOMETHYL) BENZOIC ACID METHYLESTER-SULPHATE FROM RACEMIC 4-(1-AMINOETHYL)BENZOIC ACID METHYLESTER
PROCÉDÉ DE PRÉPARATION DU SULFATE DE L'ESTER MÉTHYLIQUE D'ACIDE 4-(1-AMINOÉTHYL) BENZOIQUE (R) ET (S) À PARTIR DE L'ESTER MÉTHYLIQUE D'ACIDE 4-(1-AMINOÉTHYL) BENZOIQUE RACÉMIQUE

(30) Priorität: 28.12.2005 DE 102005062966; 06.01.2006 DE 102006001160
(43) Veröffentlichungstag der Anmeldung: 17.09.2008
(73) Patentinhaber: BASF SE, 67056 Ludwigshafen (DE)
(72) Erfinder: DITRICH, Klaus, 67161 Gönnheim (DE); WINSEL, Harald, 67134 Birkenheide (DE); MOULIN, Dominique, 67117 Limburgerhof (DE)
(86) Internationale Anmeldenummer: PCT/EP2006/069890
(87) Internationale Veröffentlichungsnummer: WO 2007/077120

(56) Entgegenhaltungen:
- EP-A1- 1 029 851
- WO-A-95/08636
- WO-A1-20/05105732
- DICKINS, R.S. ET AL.: "Synthesis, Time-Resolved Luminescence, NMR Spectroscopy, Circular Dichroism and Circularly Polarised Luminescence Studies of Enantiopure Macrocyclic Lanthanide Tetraamide Complexes" CHEMISTRY - A EUROPEAN JOURNAL, Bd. 5, Nr. 3, 1. März 1999 (1999-03-01), Seiten 1095-1105, XP002432625

## Beschreibung

Die vorliegende Erfindung betrifft ein neues Verfahren zur Verfahren zur Herstellung von (R) - und (S)- 4-(1-Ammoniumethyl)benzoesäuremethylester-sulfat.

### Stand der Technik

Die Racematspaltung von Aminen durch Enzym-katalysierte Umsetzung mit Estern ist bekannt. Kitaguchi et al. (J. Amer. Chem. Soc. 111, 3094-3095, 1989) beschreiben die Racematspaltung von Aminen mit Trifluorethylbutyrat unter Subtilisin-Katalyse. Die Enantioselektivität dieser Reaktion ist jedoch stark lösungsmittelabhäng. Selbst mit dem geeignetsten der beschriebenen Lösungsmittel (3-Methyl-3-pentanol) wird nur eine mäßige Selektivität erreicht.

In WO 91/19002 wird ein Verfahren zur chiralen Anreicherung von racemischen primären Aminen beschrieben, bei dem die Amine unter Subtilisin-Katalyse mit Ethylacetat oder Ethylbutyrat umgesetzt werden. Die dabei erzielten Enantiomerenüberschüsse sind jedoch nicht befriedigend; außerdem werden lange Reaktionszeiten von einer bis mehreren Wochen benötigt.

Gotor et al. (J. Chem. Soc. Chem. Commun. 957-958, 1988) beschreiben die enantioselektive Acylierung von 2-Amino-butan-1-ol mit Ethylacetat unter Katalyse von Schweinepankreas-Lipase (PPL). Dabei wird der verwendete Ester (Ethylacetat) auch als Lösungsmittel eingesetzt. Bei Verwendung anderer Lösungsmittel bzw. anderer Enzyme werden keine befriedigenden Ergebnisse erzielt.

Von Brieva et al. (J. Chem. Soc. Chem. Commun., 1386-1387, 1990) wird die enantioselektive Synthese von Amiden aus racemischen primären Aminen durch Umsatz mit 2-Chlorpropionat unter Katalyse von Subtilisin in Hexan oder Candida cylindracea Lipase in 3-Methyl-3-pentanol beschrieben.

WO 95/08636 beschreibt ein Verfahren zur Herstellung von optisch aktiven primären und sekundären Aminen aus den entsprechenden Racematien durch enantioselektive Acylierung in Gegenwart einer Hydrolase.

Die so dargestellten optisch aktiven Amine sind jedoch abhängig von ihrer Struktur nicht sehr lagerstabil und unterliegen weiteren Nebenreaktionen.

Es bestand daher die Aufgabe, ein Verfahren bereitzustellen, das die Herstellung von optisch aktivem 4-(1-Aminoethyl)benzoesäuremethylester in hoher Ausbeute, optischer Reinheit und in hoher Lagerstabilität gewährleistete.

### Beschreibung der Erfindung

Gegenstand der Erfindung ist ein Verfahren zur Herstellung von optisch aktivem 4-(1-Ammoniumethyl)benzoesäuremethylester-sulfat durch Umsetzen von racemischem 4-(1-Aminoethyl)-benzoesäuremethylester mit einem Acylierungsmittel in Gegenwart einer Lipase zu 4-(1-Aminoethyl)benzoesäuremethylester und anschliessendem Ausfällen von 4-(1-Ammoniumethyl)benzoesäuremethylester-sulfat durch Zugabe von Schwefelsäure.

Die für das erfindungsgemässe Verfahren geeigneten Acylierungsmittel sind bevorzugt Carbonsäureester. Besonders bevorzugt sind sogenannte aktivierte Carbonsäureester, bei denen die Säurekomponente in alpha-Position zum Carbonylkohlenstoffatom eine Heteroatomsubstitution trägt. Bevorzugt werden Methoxyessigsäureester als Acylierungsmittel eingesetzt. Die Alkoholkomponente des Carbonsäureesters besteht bevorzugt aus Alkylalkoholen mit einer Kettenlänge von 1-6 C-Atomen, die verzweigt oder unverzweigt und auch substituiert sein können. Besonders geeignetes Acylierungsmittel ist Methoxyessigsäureisopropylester.

Als Lipasen können in dem erfindungsgemässen Verfahren eine Vielzahl von Enzymen eingesetzt werden. Bevorzugt werden vor allem mikrobielle Lipasen, die beispielsweise aus Hefen oder Bakterien isolierbar sind. Besonders gut geeignet sind Lipasen aus Pseudomonas, z. B. Amano P oder die Lipasen aus Pseudomonas spec. DSM 8246 oder Candida antarctica. Die von der Firma Novozymes unter dem Namen Novozyme 435^{®} vertriebene Lipase (Lipase B aus Candida antarctica) ist besonders gut für das erfindungsgemäße Verfahren geeignet.

Das verwendete Enzym kann in nativer oder in immobilisierter Form eingesetzt werden.

Als Lösungsmittel sind generell organische Lösungsmittel geeignet. Besonders gut verläuft die Reaktion in Ethern, beispielsweise in MTBE oder THF, oder in Kohlenwasserstoffen wie Hexan, Cyclohexan, Toluol oder halogenierten Kohlenwasserstoffen wie Methylenchlorid. Die Umsetzung kann allerdings auch in Abwesenheit eines Lösungsmittels durchgeführt werden.

Die Umsetzung des Acylierungsmittels mit dem racemischen 4-(1-Aminoethyl)-benzoesäuremethylester unter Enzymkatalyse wird üblicherweise bei Raumtemperatur ausgeführt. Die Reaktionszeiten dafür betragen 1 bis 48 Stunden, bevorzugt 5-24 Stunden.

Pro Mol 4-(1-Aminoethyl)-benzoesäuremethylester wird 1 bis 2 Mol, bevorzugt 1,2 bis 1,6 Mol Acylierungsmittel zugesetzt.

Die zuzusetzende Menge an Lipase hängt von der Art der Lipase und der Aktivität der Enzympräparation ab. Die für die Reaktion optimale Lipasemenge kann leicht durch einfache Vorversuche ermittelt werden. In der Regel werden 1000 Units Lipase pro mMol Amin zugesetzt.

Der Reaktionsverlauf lässt sich leicht mit üblichen Methoden beispielsweise mittels Gaschromatographie verfolgen. Im Falle der Racematspaltung beendet man die Reaktion sinnvollerweise bei einem Umsatz von 50% des racemischen Amins. Dies geschieht in der Regel durch Entfernen des Katalysators aus dem Reaktionsraum, beispielsweise durch Abfiltrieren des Enzyms.

Durch die enantioselektive Umsetzung des racemischen 4-(1-Aminoethyl)-benzoesäuremethylester mit der Lipase entsteht aus dem einen Enantiomer das entsprechend acylierte Produkt (Amid), während das andere Enantiomer unverändert bleibt. Das nun vorliegende Gemisch aus Amin und Amid lässt sich mit üblichen Methoden leicht trennen. Gut geeignet zur Trennung des Gemisches aus Amin und Amid sind beispielsweise Extraktions- oder Destillationsverfahren. Welches Enantiomer selektiv acyliert wird ist von der Wahl der Lipase abhängig und kann leicht durch einen Vorversuch ermittelt werden; Novozyme® 435 beispielsweise acyliert selektiv den (R)-4-(1 -Aminoethyl)benzoesäuremethylester.

Nach erfolgter Herstellung von optisch aktivem 4-(1-Aminoethyl)benzoesäuremethylester wird dieser durch Zugabe von Schwefelsäure in optisch aktives 4-(1-Ammoniumethyl)benzoesäuremethylester-sulfat überführt. Pro mol 4-(1-Aminoethyl)benzoesäuremethylester werden mindestens 0.5 mol Schwefelsäure zu der Reaktion gegeben, um eine vollständige Salzbildung (Überführung in 4-(1-Ammoniumethyl)benzoesäuremethylester-sulfat) zu gewährleisten.

Die Zugabe von Schwefelsäure kann direkt an die enzymatische Acylierung erfolgen oder aber nach vorangehender Isolierung von 4-(1-Aminoethyl)benzoesäuremethylester aus dem Reaktionsmedium. Man kann auch nur die Lipase aus dem Reaktionsmedium entfernen; dies wird besonders vorteilhaft dann angewendet, wenn mit immobilisierter Lipase gearbeitet wird.

In der Regel fällt das 4-(1-Ammoniumethyl)benzoesäuremethylester-sulfat aus dem Reaktionsmedium aus und kann somit leicht vom gelösten acylierten 4-(1-Aminoethyl)-benzoesäuremethylester (d.h. dem Amid) abgetrennt werden. Falls beide Reaktionsprodukte gelöst vorliegen, können sie leicht aufgrund ihrer verschiedenen physikochemischen Eigenschaften durch Standardoperationen wie Kristallisation, Extraktion, Destillation und Chromatographie getrennt werden.

4-(1-Ammoniumethyl)benzoesäuremethylester-sulfat ist unter den Reaktionsbedingungen stabil und erfährt keine intermolekulare Amidierung wie sie beispielsweise bei 4-(1-Aminoethyl)benzoesäuremethylester beobachtet wird. 4-(1-Ammoniumethyl)-benzoesäuremethylester-sulfat kann durch weitere Reinigung wie Umkristallisieren in hoher optischer Reinheit erhalten werden.

### Experimenteller Teil

### Beispiel 1

### Herstellung von (S)-4-(1-Ammoniumethyl)-benzoesäuremethylester-sulfat (S-1-Sulfat)

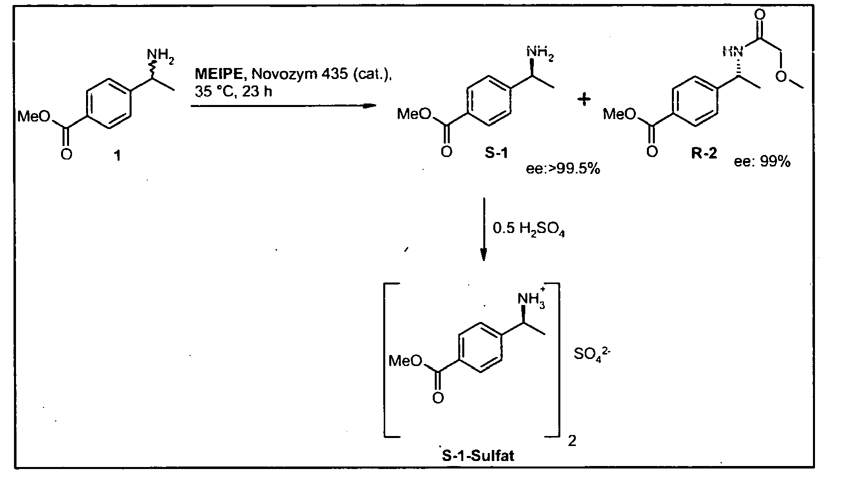

### Durchführung:

Eine Lösung von 4-(1-Aminoethyl)-benzoesäuremethylester 1 (17.9 g, 0.1 mol) in Toluol (150 mL) wurde mit Methoxyessigsäure-isopropylester **MEIPE** (19.8 g, 0.15 mol) und Novozym 435 (500 mg) versetzt und bei Raumtemperatur gerührt. Nach 24 Stunden war It. HPLC-Analytik alles **R-1** zum R-Amid **R-2** umgesetzt und nur noch unumgesetztes S-Amin **S-1** nachweisbar. Das Enzym wurde über Kieselgur abgesaugt, der Filterrückstand mit Toluol (20 mL) gewaschen und das Filtrat unter Rühren mit Schwefelsäure (6.45 g einer 38%-igen Lösung in Wasser, 25 mmol) versetzt. Dabei fiel ein weißer, voluminöser Niederschlag aus. Man saugte das ausgefallene Salz ab, wusch mit Toluol (2 x 20 mL) und kristallisierte den Filterrückstand aus Wasser (10 mL) um. Man isolierte 8.9 g (78%) Sulfat **S-1** als weißen, kristallinen Feststoff, das darin gebundene S-Amin **S-1** war It. HPLC-Analytik enantiomerenrein. Schmelzpunkt: 280 °C (Zersetzung), Drehwert: [α]_{D}= - 3.5° (c=1 in H₂O).

Die vereinten Toluol-Filtrate wurden noch einmal mit Wasser (10 mL) gewaschen, über Natriumsulfat getrocknet und eingeengt. Der verbliebene Rückstand wurde im Ölpumpenvakuum bei 50 °C von restlichem Lösungsmittel befreit. Man behielt 11 g (88%) R-Amid **R-2** als schmierigen Feststoff (Fp.: 50 - 60 °C) zurück, das It. GC-Analytik zu 90% rein war.
¹H-NMR-Spektren:

### Amin 1:

¹H-NMR (400 MHz, CDCl₃) δ= 1.40 (d, J= 7 Hz, 3H); 1.60 (s, breit, 2H), 3.90 (s, 3H), 4.15 (q, J= 7 Hz, 1 H), 7.45 und 8.03 (AA, BB -System, J_{AB}= 10.7 Hz, 4H).

### S-Amin-Sulfat S-1 -Sulfat:

¹H-NMR (400 MHz, D₂O) δ= 1.65 (d, J= 7 Hz, 3H); 3.95 (s, 3H), 4.65 (q, J= 7 Hz, 1H), 7.60 und 8.10 (AA', BB-System, J_{AB}= 10.7 Hz, 4H).

### R-Amid R-2:

¹H-NMR (400 MHz, CDCl₃) δ= 1.55 (d, J= 7 Hz, 3H); 3.42 (s, 3H), 3.88 und 3.93 (AB-System, J_{AB}= 15 Hz, 2H), 3.90 (s, 3H), 5.23 (sept, J= 7 Hz, 1 H), 6.80 (d (breit), J= 7 Hz, 1 H), 7.40 und 8.03 (AA', BB -System, J_{AB}= 10.7 Hz, 4H).

## Patentansprüche

1. Verfahren zur Herstellung von optisch aktivem 4-(1-Ammoniumethyl)-benzoesäuremethylester-sulfat durch Umsetzen von racemischem 4-(1-Aminoethyl)-benzoesäuremethylester mit einem Acylierungsmittel in Gegenwart einer Lipase zu 4-(1-Aminoethyl)benzoesäuremethylester und anschliessendem Ausfällen von 4-(1-Ammoniumethyl)benzoesäuremethylester-sulfat durch Zugabe von Schwefelsäure.

2. Verfahren nach Anspruch 1, **dadurch gekennzeichnet, dass** als Acylierungsmittel ein Methoxyessigsäureester verwendet wird.

3. Verfahren nach Anspruch 1, **dadurch gekennzeichnet, dass** als Lipase Novozyme^{®} 435 verwendet wird.

4. Verfahren nach Anspruch 1, **dadurch gekennzeichnet, dass** die Umsetzung in einem Lösungsmittel durchgeführt wird.

5. Verfahren nach Anspruch 1, **dadurch gekennzeichnet, dass** vor Zugabe der Schwefelsäure das Enzym aus dem Reaktionsmedium entfernt wird.

6. (S)-4-(1-Ammoniumethyl)benzoesäuremethylester-sulfat

7. (R)-4-(1-Ammoniumethyl)benzoesäuremethylester-sulfat

## Claims

1. A method for preparing optically active methyl 4-(1-ammonioethyl) benzoate sulfate by reacting racemic methyl 4-(1-aminoethyl)benzoate with an acylating agent in the presence of a lipase to give methyl 4-(1-aminoethyl) benzoate and subsEquently precipitating methyl 4-(1-ammonioethyl)benzoate sulfate by adding sulfuric acid.

2. The method according to claim 1, wherein a methoxyacetic ester is used as acylating agent.

3. The method according to claim 1, wherein Novozyme^{®} 435 is used as lipase.

4. The method according to claim 1, wherein the reaction is carried out in a solvent.

5. The method according to claim 1, wherein the enzyme is removed from the reaction medium before addition of the sulfuric acid.

6. Methyl (s) -4-(1-ammonioethyl) benzoate sulfate

7. Methyl (R)-4-(1-ammonioethyl) benzoate sulfate

## Revendications

1. Procédé pour la préparation de sulfate d'ester méthylique de l'acide 4-(1-ammonioethyl)-benzoïque optiquement actif par transformation d'ester méthylique de l'acide 4-[1-aminoéthyl)-benzoïque racémique avec un agent d'acylation en présence d'une lipase en ester méthylique de l'acide 4-(1-aminoéthyl) benzoïque et précipitation consécutive de sulfated de l'ester méthylique de l'acide 4-(1-ammonioéthyl)benzoïque par addition d'acide sulfurique

2. Procédé selon la revendication 1, **caractérisé en ce qu'**on utilise un ester de l'acide méthoxyacétique comme agent d'acylation.

3. Procédé selon la revendication 1, **caractérisé en ce qu'**on utilise comme lipase le Novozyme® 435.

4. Procédé selon la revendication 1, **caractérisé en ce qu'**on réalise la transformation dans un solvant

5. Procédé selon la revendication 1, **caractérisé en ce que** l'enzyme est éliminée du milieu réactionnel avant l'addition de l'acide sulfurique.

6. Sulfate de l'ester méthylique de l'acide (S)-4-(1-ammonioéthyl)benzoïque.

7. Sulfate de l'ester méthylique de l'acide (R)-4-(1-ammonioéthyl)benzoïque.
